# EUROPEAN PATENT APPLICATION

(11) **EP 4 272 661 A1**
(43) Date of publication of application: **08.11.2023**
(21) Application number: 22315099.6
(22) Date of filing: 06.05.2022
(51) Int. Cl.: A61B 17/34, A61M 39/06, A61B 17/00

(54) **SURGICAL ACCESS PORT AND SYSTEM COMPRISING A SURGICAL ACCESS PORT**

(71) Applicant: Caranx Medical SAS, 75013 Paris (FR)
(72) Inventor: BERTHET-RAYNE, Pierre, 06800 Cagnes-sur-Mer (FR); SMITS, Jonas Victor Hamen, 3360 Bierbeek (BE); SEJOR, Eric, 06000 Nice (FR); MIALHE, Claude, 13100 Le Tholonet (FR)
(74) Representative: Hepp Wenger Ryffel AG

(57) **Abstract**

The invention relates to a surgical access port (101) for introduction of surgical instruments (11) into a pressurized anatomical region (12). The surgical access port (101) comprises an elongated lumen (1) and at least one sealing unit (2) placed or placeable within the lumen (1). The sealing unit (2) comprises at least one sealing element (21, 22) which is convertible from an open configuration (301) to a closed configuration (303) and vice versa. In the closed configuration (303) an aperture (211, 221) of the sealing element (21, 22) is closed radially inward to a smaller cross-section (4), preferably a cross-section of 0.0 mm to render the lumen (1) pressure tight. In the open configuration of the sealing element (21, 22) the aperture (211, 221) has a maximum cross-section (41), such that fluid communication is established between a proximal side (34) of the sealing element (21, 22) and a distal side (36) of the sealing element (21, 22). The sealing element (21, 22) comprises an intermediate configuration (302) between the open configuration (301) and the closed configuration (303).

## Description

The invention relates to a surgical access port, system and method for introduction of surgical instruments into a pressurized anatomical region according to the independent claims.

Endovascular, natural orifice, single port or laparoscopic surgical procedures often require the insertion, exchange, and removal of surgical instruments of varying diameters into an often pressurized anatomy through a surgical access port.

Various access ports for inserting surgical instruments are known in the prior art. Surgical access ports with haemostatic valves often comprise a passive sealing structure through which the insertion of surgical instruments has to be carried out manually when performing endovascular surgery in an artery.

US 2013/331655 A1 discloses an access port and sheath assembly comprising a hemostatic valve with four valve segments of a resilient material for preventing leakage of blood during insertion of surgical instruments.

WO2006/113800 A2 discloses a self-sealing surgical access port with an elastic membrane being biased to form a constricted region within a tube by default which is elastically deformable radially outwardly to permit surgical tools to pass through.

EP 1 833 550 A1 discloses a valve for a surgical or medical instrument which can be at least partially closed by actuation means comprising a cable for actuating a rotation of a mobile end to generate torsion.

However, there is generally a lack of a simple, intuitive, user-friendly, and fast insertion, exchange, and removal of surgical instruments, in particular with different cross-sections, through a single access port.

In addition, a passive sealing structure of a surgical access port comprising resilient material often exerts high friction on the surgical instruments which can impair functionality and controllability of the surgical instruments.

Passive sealing structures further only allow for a limited range of adaptation of diameters.

Furthermore, known access ports often lack automation and/or remote controlling capabilities of the access port for adjusting to different instrument sizes without resulting in leakage.

It is the object of the present invention to overcome these and other disadvantages of the prior art. In particular, the invention shall provide a simple access port for introduction of surgical instruments with different cross-sections into a pressurized anatomical region shall provide improved handling of the surgical instruments, and operability of the aperture/s, and preferably automated and/or remote-controlled operability.

The objects are solved by an access port, a method for operating an access port, and a system as defined in the independent claims. Further embodiments result from the dependent claims.

A surgical access port according to the invention is used for the introduction of surgical instruments into a pressurized anatomical region. It comprises an elongated lumen and at least one sealing unit placed or placeable within the lumen. The sealing unit comprises at least one sealing element which is convertible from an open configuration to a closed configuration and vice versa. In the closed configuration an aperture of the sealing element is closed such that it has a radially inward reduced cross-section, preferably a cross-section of 0.0 mm², to render the lumen pressure tight. In the open configuration of the sealing element, the aperture has a maximum cross-section such that fluid communication is established between a proximal side of the sealing element and a distal side of the sealing element. The sealing element comprises an intermediate configuration between the open configuration and the closed configuration. In the intermediate configuration, the cross-section of the aperture is configured to receive surgical instruments with a particular cross-sectional dimension, corresponding to the cross-section of the aperture, to be inserted through the sealing unit into the pressurized anatomical region, rendering the sealing element pressure tight. The intermediate configuration can be reached actively and independently, i.e. without the need of an instrument.

Pressure tightness in the context of the sealing element is de-, fined as the pressure tightness to at least the prevailing blood pressure in a patient's pressurized anatomical regions.

The aperture of the sealing element can be arranged coaxially relative to the lumen of the surgical access port. When the sealing element is converted from an open to a closed state, the opening of the sealing element can be closed substantially centrally radially inwardly around an axis of symmetry of the lumen.

The lumen may be substantially uniformly cylindrical in shape along its longitudinal axis.

The sealing element may be recessed into the surgical access port so that the aperture of the sealing element in the open configuration has a cross-section corresponding to the neighbouring cross-section of the lumen.

The sealing element in the open configuration can assume an at least partially cylindrical shape, essentially parallel to a longitudinal direction of the lumen.

The sealing element in the intermediate and/or closed configuration can form an at least partially, preferably completely, hourglass, circular conical, or elliptic conical shape, preferably around the symmetry axis of the lumen. Such a shape enables easy and reliable positioning of surgical instruments within the sealing element.

The aperture can be closed and/or opened uniformly in the radial direction of the lumen. This ensures uniform sealing and enclosing of surgical instruments and the lumen.

The maximum cross-section of the aperture can be 25%, in particular 20%, in particular preferably 15% less, than the cross-section of a subsection of the lumen in which the aperture is placed. Such a large maximum cross-section of the aperture allows versatile use of the surgical access port for multiple surgical instruments with different cross-sectional dimensions without unduly increasing a cross-sectional dimension of the surgical access port.

The cross-section of the aperture of the intermediate configuration can preferably be adjusted, between at least two different cross-sections other than the cross-section of the open configuration or the closed configuration.

The cross-section of the aperture in the intermediate configuration can be continuously adjustable between the open configuration and the closed configuration. Thus, surgical instruments of any cross-sectional dimensions can be accommodated in a pressure tight manner.

The sealing unit can be configured to assume the closed configuration in a default state without any external force being applied. This is advantageous, as it ensures the safety of a patient in the event of a defect in the surgical access port, in particular a defect of the control of the sealing unit.

The sealing unit can be configured for receiving a surgical instrument with a particular cross-sectional dimension, such that the surgical access port is sealed but at the same time the friction of moving the surgical instrument in longitudinal direction of the surgical access port is minimized.

The sealing element can comprise a proximal mount, a distal mount, and a closing element, in particular a closing element formed as a bellows. The proximal mount and the distal mount can be connected to the closing element. The closed configuration can be converted via the intermediate configuration to the open configuration of the sealing element by a relative movement of one of the proximal mount and the distal mount relative to the other of the proximal mount and the distal mount.

The sealing element or parts of the sealing element, in particular the proximal mount and/or distal mount, can be continuously connected or connectable along the boundary of the lumen in a circumferential direction.

The proximal mount and/or distal mount can have an at least partially hollow conical or hollow cylindrical shape, in particular with a circular or elliptic base.

In the open configuration, the proximal mount and distal mount may be spaced farthest apart from each other, in particular in the longitudinal direction of the surgical access port. In the closed configuration the proximal mount and the distal mount can be arranged closest together.

The proximal mount and the distal mount may be spaced apart so that they do not contact each other regardless of which configuration the sealing element is in.

The surgical access port may include an elastic member, particularly a spring, that expands or contracts the proximal mount or distal mount along the axis of the lumen. By means of such a spring, it can be ensured that the sealing element is in a default state in the closed position or open configuration. Furthermore, due to the restoring force of an elastic member, a one directional actuation for the relative movement between the proximal mount and distal mount in at least two directions, e.g., along the longitudinal direction of the surgical access port, can be sufficient.

The relative movement of the proximal mount and/or distal mount can be one of the following:
- A relative movement in a longitudinal direction of the surgical access port,
- A relative movement in a rotational direction, preferably in a circumferential direction of the lumen of the surgical access port, and
- A relative movement in the longitudinal direction of the surgical access port and in the rotational direction, preferably in the circumferential direction of the lumen of the surgical access port.

The above-mentioned relative movement of the proximal mount and/or the distal mount allows a simple and adjustable conversion of the sealing element from the open configuration to the closed configuration and vice versa.

A relative movement in the longitudinal direction is particularly easy to implement mechanically.

The proximal mount or the distal mount can be immovably arranged within the lumen of the surgical access port. Alternatively, both the proximal mount and distal mount can be movable, in particular in only the longitudinal or the circumferential direction of the lumen, within the lumen.

Immovably arranging the proximal mount within the lumen has the advantage that the relative movement of the distal mount in the longitudinal direction of the access port for sealing the sealing element is directed toward a proximal end of the surgical access port, and thus away from the patient. Thereby, the sealing properties can be optimized and possible backflow of blood prevented.

If both proximal and distal mount are movable relative to the lumen, in particular in only the longitudinal or the circumferential direction of the lumen, the proximal and distal mount can be configured to be movable along opposite longitudinal directions. The proximal and distal mounts can be'moved with the same force over the same distance in opposing directions to achieve the conversion of the sealing element. Thereby, the sealing element can be uniformly and symmetrically converted to the closed configuration.

Such a symmetrical relative movement of the proximal and distal mount may cancel at least a part of the frictional forces which could be exerted on a surgical instrument inside the lumen. Thus, unwanted relative movement between the surgical instrument and the surgical access port can be avoided and the surgical instrument can be enclosed by the sealing unit to remain static.

The closing element can comprise or consist of a flexible material such as thermoplastic material, such as silicone, latex; polyurethane, polyethylene, polyamide, polyethylene terephthalate, polyvinyl chloride, polyether block amide and preferably forms a continuous at least partially tubular structure.

Such a material has the advantage, that the closing element can be elastically deformed, in particular torsionally deformed, by the relative movement between the proximal and distal mount, in particular relative rotation between the proximal and distal mount.

The closing element comprising or consisting of a less stretchable flexible material minimizes the risk of defects due to plastic deformations.

By using flexible materials for the closing element, a stronger sealing effect can be achieved on the surgical instrument or the opposite side of the closing element when closing the aperture radially inwardly. Due to the flexible material being more compliant the sealing effect may be increased by adapting the shape of the closing element better to the surface against which it is pressed and by forming a more continuous and larger sealing contacting surface of the closing element with the surgical instrument or with itself. Thus, the pressure tightness of the sealing element can be improved.

It is, however, also possible to use a closing element in the form of a mechanism with a diaphragm, as used e.g. in optics.

The closing element can be fixedly connected to the proximal and/or distal mount or rotatably connected relative to the proximal and/or distal mount.

A closing element which is fixedly attached to the proximal mount and the distal mount can be configured to be twistable by the rotation of at least one of the proximal and distal mount relative to each other, such that the closing element forms a constricted section in the middle of the sealing element. The closing element can then be torsionally deformable. Thereby multiple twists of a part of the closing element relative to a different part, e.g., the distal part relative to a proximal part, of the closing element can ensure pressure tightness of the sealing unit.

The closing element may be shaped, texturized or prefolded such that that the relative movement converts the closing element to a predefined geometric shape. In the predefined geometric shape, the surface of the closing element can interlock particularly well and consistently so that pressure tightness is optimized.

The closing element can have a varying material thickness, in particular along the longitudinal direction of the surgical access port. Thereby, the durability and the sealing property, in particular with regard of multiple repeated opening and closing cycles, of the sealing element can be increased.

The surgical access port can comprise a guiding structure for guiding at least one of the proximal mount and the distal mount during the relative movement. The guiding structure can comprise an abutment or stop member which causes an increased resistance against the relative movement in at least a partial area of the guiding structure.

The guiding structure may include a radial projection or recess.

The guiding structure can form a rail-shape or a thread-shape in a helical, the longitudinal, or the circumferential direction of the surgical access port.

An increased resistance against the relative movement due to the abutment or stop member of the guiding structure can provide a user or controller with feedback at which partial area of the guiding structure the mount is located. In addition, the increased resistance can help to retain a certain configuration of the sealing element more reliably, without providing active forces, e.g. holding currents of motors.

Multiple abutment or stop members can be arranged at multiple areas, preferably equally distant areas, of the guiding surface.

Certain abutment or stop members can correspond to certain cross-sections of the aperture and thus to certain cross-sectional dimensions of surgical instruments.

The sealing unit can comprise a first sealing element and a second sealing element which are spaced apart from each other in the longitudinal direction of the surgical access port.

Multiple sealing elements have the advantage of improving the tightness of the surgical access port and allow two-stage active adjustment of the sealing elements, so that one sealing element is always pressure-tight.

The first sealing element and the second sealing element can be convertible from the closed configuration to the open configuration via the intermediate configuration and vice versa independently from each other.

Independently controlling the first and second sealing element reduces the risks of leakage. While the second distal sealing element can remain fully closed, the proximal first sealing element can be configured to a specific cross-section e.g., to the cross-sectional dimensions of a surgical instrument, and vice versa.

The sealing unit can have an actuation element for operating the at least one sealing element. The actuation element can be a pneumatic, a hydraulic, an electrical, magnetic, electromagnetic, electromechanical, or a mechanical.actuation element.

The actuation element can be connected or connectable to at least one of the proximal mount, the distal mount, and the closing element in order to actuate the relative movement between the proximal and distal mount.

The actuation element may also comprise a pull wire.

The magnetic actuation element may comprise an electromagnet, in particular a solenoid, or a permanent magnet which is arranged at least partially coaxially around at least one sealing element, preferably without contacting the sealing element.

The proximal and/or distal mount can comprise or consist of a ferromagnetic core or a permanent magnet such that the sealing element can be operated by the magnetic actuation element.

The magnet of the magnetic actuation element and a permanent magnet of the proximal and/or distal mount may be aligned or alignable with equal or opposite polarity of the magnets relative to each other to actuate the relative movement.

The guiding structure for guiding of the proximal mount and/or distal mount during relative movement can be arranged within the lumen of the surgical access port. Thereby, the external actuation by the actuation element can be configured to relatively move the proximal or distal mount in a helical motion due to the optionally threaded/slotted guiding structure within the surgical access port.

The magnetic actuation element can be configured to generate an essentially longitudinal field vector or helical field vector to exert a force on the magnet or ferromagnetic material of the proximal or distal mount.

At least a part of the actuation element, in particular the magnet of the actuation element, can be arranged on an actuation guiding structure for guiding at least a part of the actuation element. The actuation guiding structure can be arranged on the outer surface of the surgical access port and can form a thread-shape or slots.

The actuation element may include a mechanical transmission such that a rotational motion is convertible to a linear motion or vice versa. By means of such a transmission, the actuation force for carrying out the relative movement can be exerted in a targeted direction and the arrangement of the actuation element can be optimized.

The sealing element can alternatively comprise or be associated with a volume adjustment unit for operating the sealing element by adjusting the volume of a fluid in at least one pressure tight fluid chamber of the volume adjustment unit. The volume adjustment unit can further comprise at least one fluid reservoir fluidly connected or fluidly connectable to the at least one fluid chamber by decrease/increase of the pressure.

Pressure tightness with respect to the fluid chamber is defined by sufficient pressures for the intended use of converting the sealing element from the open configuration to the closed configuration or vice versa.

The volume adjustment unit can be configured to function with a pressure increase by introducing fluid into the fluid chamber or a pressure decrease by removing fluid from the fluid chamber.

The fluid within the volume adjustment unit can be prefilled and/or be a bio compatible substance, in particular saline solution.

The fluid chamber can comprise a flexible material such as stretchable elastomers or other thermoplastic material, such as silicone.

The sealing element comprising a volume adjustment unit can in addition comprise a movable proximal or distal mount. The closing element can be formed by the fluid chamber. Thereby, both a relative movement between the proximal and distal mount and the adjustment of a fluid volume in the pressure tight fluid chamber can enhance the sealing properties of the surgical access port.

The volume adjustment unit can comprise at least two independently controllable valves to operate at least two sealing elements.

The two independently controllable valves may be fluidly connected to a common fluid reservoir or separate fluid reservoirs.

The surgical access port can comprise an outer monolithic tubular member. The monolithic tubular member can form a substantially continuous surface.

A monolithic tubular member which encloses the lumen without a cut-out or a recess e.g., for receiving the actuation element and/or sealing unit, is easier to manufacture and ensures tightness against production defects.

The actuation element can be arranged at least partially or completely, inside the lumen or enclosing the tubular member. An actuation element which at least partially encloses the tubular member is not limited by the dimensions of the lumen and can also be accessed or replaced in case of a defect.

The closing element of the sealing element can be spaced apart from the tubular member.

A system comprising a previously described surgical access port can further comprise a controller configured to operate the at least one sealing element of the sealing unit, preferably at least two sealing elements independently from each other.

The controller can be configured for converting the sealing elements from the closed configuration to the intermediate configuration and the open configuration and vice versa.

The controller can be configured for remote operation of the sealing elements, preferably fully automated, without the need of a user input. Such a configuration of the controller has the advantage that a surgeon can fully focus on surgical intervention without devoting attention on the introduction of surgical instruments.

The system can comprise a sensor for detecting the pressure exerted on a surgical instrument within the aperture by the sealing element.

The sensor can additionally or alternatively be adapted for detecting if a surgical instrument is inserted into the proximal section of a sealing element of the surgical access port and/or if the surgical instrument contacts or is pressed against the sealing element.

The surgical access port, in particular the sealing unit, can comprise an optical, capacitive, resistive, ultrasound, or pressure sensor for detecting the attempt of an insertion of a surgical instrument through the aperture in a closed configuration.

The controller can be configured, in particular by operating the actuation element, to adjust the cross-section of at least one sealing element, optionally based on the detected data of a sensor.

The controller can be configured to adjust the cross-section of the at least one sealing element to at least one, in particular two different, pre-determined cross-sections, preferably corresponding to at least one, in particular two, cross-sectional dimensions of surgical instruments.

The sealing element may be configured to detect an attempt of insertion of a surgical instrument through the aperture in the closed configuration.

The sealing element may be configured to detect a radial force due to insertion of the surgical instrument through the aperture and to adjust pressure after insertion of the surgical element so as not to apply an unnecessarily increased friction to the surgical instrument.

The system can comprise an instrument inserter for engaging the surgical instrument and actuating an insertion of the surgical instrument through the surgical access port along the longitudinal direction of the surgical access port. Such a system can optimize the temporal and spatial coordination of the sealing unit and the instrument positioner with respect to the surgical instrument when inserting the surgical instrument by the instrument inserter.

The instrument inserter may be operatable by the controller.

A system according to the invention can comprise a previously described surgical access port and at least one surgical instrument, preferably at least two surgical instruments with at least two different cross-sectional dimensions.

Instruments can typically be catheters and guidewires for transcutaneous implantation of implants such as in the TAVI procedure. The implants may be, for example, a stent, a graft, or a prosthetic valve replacement.

A method for introduction of surgical instruments of different cross-sectional dimensions into a pressurized anatomical region through the surgical access port, in particular as described above, comprises the steps of:
- Providing a second sealing element in a closed configuration;
- Optionally converting a first sealing element from a closed configuration to an intermediate configuration or an open configuration;
- Introducing the surgical instrument from a proximal side to an intermediate section of the surgical access port through a first aperture of the first sealing element;
- Converting the first sealing element from the intermediate configuration or open configuration such that the cross-section of the first aperture encloses the surgical instrument in a pressure tight manner;
- Optionally, converting the second sealing element from the closed configuration to the intermediate configuration or open configuration;
- Introducing the surgical instrument from the intermediate section to the distal side of the surgical access port through a second aperture of the second sealing element.
- Converting the second sealing element from the intermediate configuration or open configuration such that the cross-section of a second aperture encloses the surgical instrument in a pressure tight manner.

The method can comprise the step of engaging, in particular frictionally engaging a surgical instrument such as a guidewire within the sealing unit to hold the guidewire in place with respect to the surgical access port, such that surgical instruments can be advanced over the fixedly held guidewire.

The invention is now described with reference to certain embodiments and figures which'show:
Figure 1A: a longitudinal section of a first embodiment of the surgical access port with a sealing unit;
Figures 1B to 1E: a longitudinal section of a sealing unit according to Fig. 1A in a different configuration;
Figures 2A and 2B: a longitudinal section of a second embodiment of a surgical access port with a sealing unit having two sealing elements;
Figures 2C and 2D: a longitudinal section of the second embodiment of the surgical access port according to Fig. 2A and 2B with inserted surgical instruments;
Figure 3: a longitudinal section of a third embodiment of a surgical access port with a sealing unit;
Figure 4: a longitudinal'section of a fourth embodiment of a surgical access port with a sealing unit;
Figures 5A to 5F: a longitudinal section of the embodiment of the surgical access port according to Fig. 2A and 2B during the insertion of a surgical instrument;
Figure 6: a schematic arrangement of a system comprising a surgical access port connected to a pressurized anatomical region of a patient.

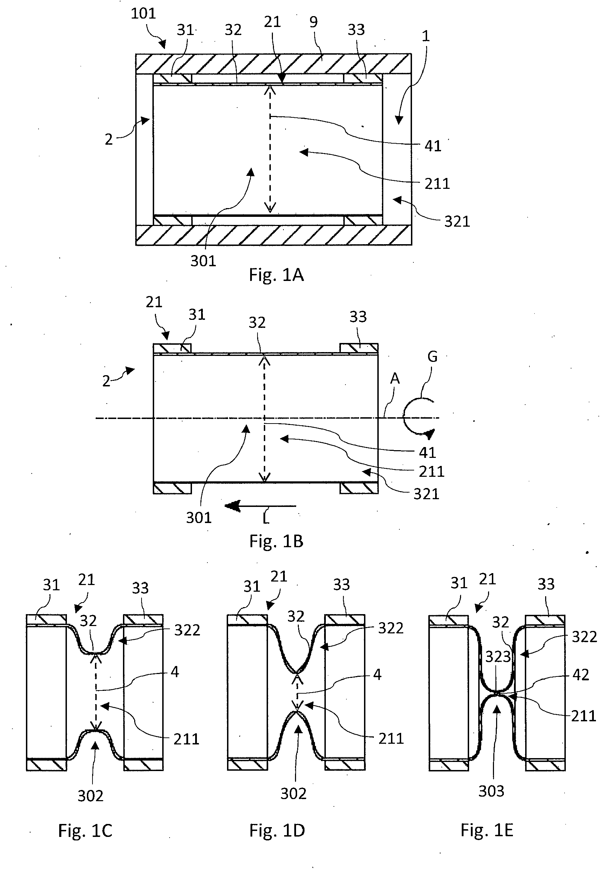

Figure 1A shows a longitudinal section of a first embodiment of a surgical access port 101 with a lumen 1. A sealing unit 2 with a single sealing element 21 which is arranged within the lumen 1. The sealing element 21 has a proximal mount 31 and a distal mount 33 which are both connected to a membrane 32. The proximal mount 31 and the distal mount 33 have a hollow cylindrical shape and are circumferentially connected to the tubular member 9 of the surgical access port 101. The membrane 32 is fixedly attached on the inner surface of the cylindrically shaped proximal mount 31 and distal mount 33. However, the membrane 32 is not connected to the tubular member 9 itself.

The sealing element 21 of Fig. 1A is in an open configuration 301 such that the flexible membrane 32 forms a coaxially tubular shape 321 coaxially to the lumen 1 with an aperture 211 with a maximum cross-section 41.

The proximal mount 31 is immovably connected to the tubular member 9. The proximal mount 31 may also be materially bonded with the tubular member 9.

The distal mount is movably connected to the tubular member 9 such that the proximal mount 31 and the distal mount 33 are movable relative to each other. The proximal mount 31 and distal mount 33 in the open configuration 301 in Fig. 1A are spaced apart in the longitudinal direction such that the membrane 32 assumes a stretched state with a maximum longitudinal extension.

Figures 1B to 1E show a longitudinal section of a sealing unit 2 according to the embodiment of Fig. 1A. The tubular member was omitted for clarity. The sealing unit 2 is depicted in different configurations 301, 302, 303. Fig. 1B shows a maximum aperture 211 of the sealing element 21 in an open configuration 301. The open configuration 301 can be converted to an intermediate configuration 302 (Fig. 1C and Fig. ID) by moving the distal mount 33 closer to the proximal mount 31.

The tubular member may be actuated such that the distal mount 33 moves closer to the proximal mount 31 by a relative rotational movement between the tubular member and the distal mount 33 while maintaining a coaxial relationship between the proximal mount 32 and the distal mount 33. The relative movement of the distal mount 33 is in a rotational direction G circumferential to the surgical access port 101 which leads to a longitudinal movement component in longitudinal direction L of the surgical access port 101. By performing such a relative rotational movement, the membrane 32 is compressed and twisted at the same time, so that the aperture 211 can be closed continuously in a radially inward direction which decreases its cross-section 4. Due to the relative movement, the membrane 32 assumes a partially folded hourglass shape 322 in Fig. 1C and Fig. 1D. Thereby, the aperture 211 in the intermediate configurations 302 shown in Fig. 1C and Fig. 1D can be continuously decreased in size until the cross-section 4 of the aperture 211 is completely sealed with at a minimum cross-section 42 in a closed configuration 303 shown in Fig. 1E. Once the closed configuration 303 of Fig. 1E is reached, a contacting surface 323 of the closing element 2 may still be increased by further relative rotational movement of the distal mount 33 to enhance the sealing properties of the sealing element 21 by additional twisting of the membrane 32.

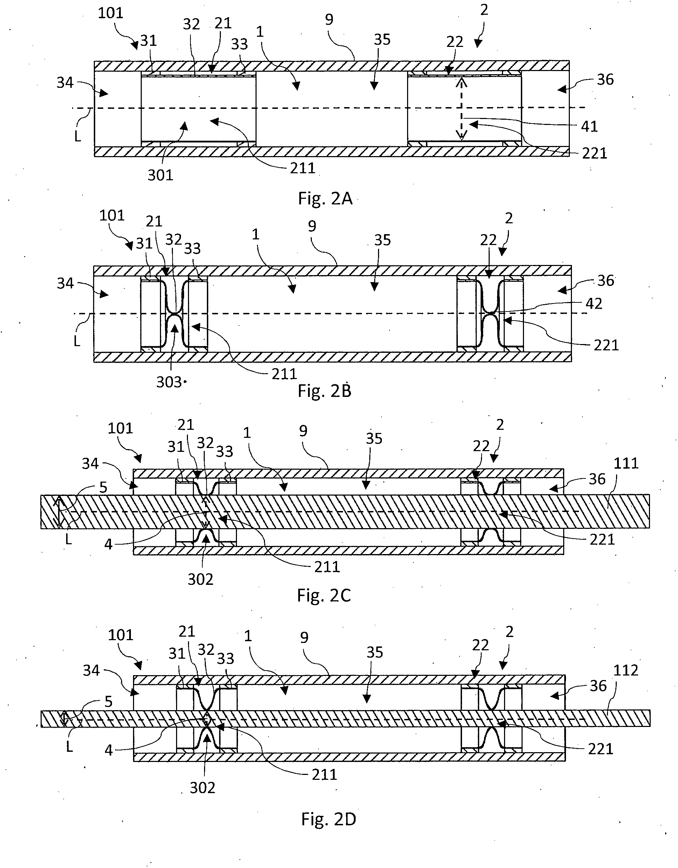

Figures 2A and 2B show a longitudinal section of a second embodiment of the surgical access port 101 with a sealing unit 2 having of two sealing elements 21, 22 which are arranged offset from each other in a longitudinal direction L of the surgical access port 101.

The first sealing element 21 and the second sealing element 22 in Fig. 2A and 2B are otherwise structurally identical and each comprises a proximal mount 31 and distal mount 33 connected to a membrane 32 analogous to Fig. 1A - Fig. 1E.

In Fig. 2A both sealing elements 21, 22 are in an open configuration 301 such that the apertures 211, 221 have a maximum cross-section 41.

In Fig. 2B both sealing elements 21, 22 are in a closed configuration 303 such that apertures 211, 221 are completely closed and have a minimum cross-section 42. In the open configuration 301 in Fig. 2A a fluid, communication between a proximal side 34 and a distal side 36 is established whereas in a closed configuration 303 in Fig. 2B the proximal side 34, an intermediate section 35, and the distal side 36 are sealed off from each other by the sealing elements 21, 22.

Figures 2C and 2D show a longitudinal section of the second embodiment of the surgical access port 101 according to Fig. 2A and 2B with an inserted surgical instrument 111, 112 into a lumen 1 of the surgical access port 101. The first surgical instrument 111 in Fig. 2C has a greater cross-sectional dimension 5 than the second surgical instrument 112 in Fig. 2D. The sealing elements 21, 22 are adapted to adjust the cross-section 4 of the aperture 211, 221 in the intermediate configuration 302 such that the surgical elements 111, 112 can be received without causing strong friction with the membrane 32. Therefore, the surgical instruments 111, 112 are movable in a longitudinal direction L of the surgical access port 101 with a minimum frictional resistance.

The sealing elements 21, 22 can be converted analogously to Fig. 1A to 1E by relative movement between the distal mount 33 and the proximal mount 31. At the same time the pressure tightness against fluid of the proximal side 34, the intermediate section 35, and the distal side 36 can be ensured by the enclosure between the membrane 32 and the surgical instruments 111, 112. The cross-section 4 of the apertures 211, 221 in Fig. 2C and Fig. 2D can either be adjusted prior to the insertion of the surgical instruments 111, 112 or after the insertion of the surgical instruments 111, 112 by detecting the cross-sectional dimension 5 or a detection of an exerted force.

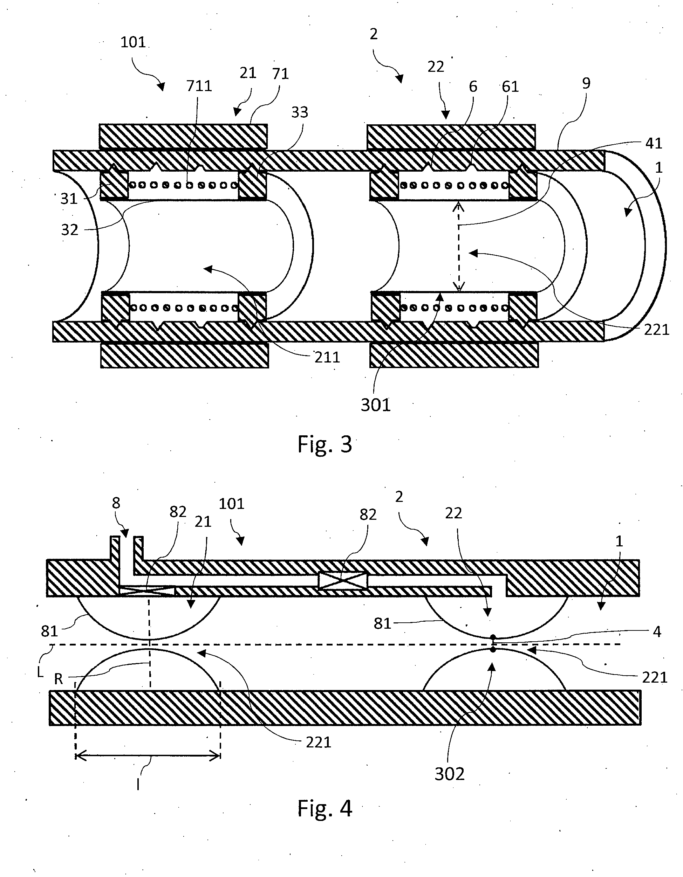

Figure 3 shows a perspective cross-section of a third embodiment of a surgical access port 101 with a sealing unit 2 having two sealing elements 21, 22. The sealing elements 21, 22 have a proximal mount 31 and a distal mount 33 which are connected to a membrane 32. The membrane 32 may be configured to assume an essentially cylindrical shape in an open configuration 301 and a radially inward convex shape in an intermediate and a closed configuration.

Both sealing elements 21, 22 in Fig. 3 are in the open configuration 301 such that the apertures 211, 221 have a maximum cross-section 41.

A tubular member 9 enclosing a lumen 1 is monolithic such that the sealing elements 21, 22 have to be actuated without a physical contact through the tubular member 9. This can be achieved by coaxially arranged solenoids 71 which are arranged circumferentially around the tubular member 9 to actuate the sealing elements 21, 22. The solenoids 71 are configured to generate an essentially longitudinal field vector to exert a force on the distal mounts 33 which comprise a ferromagnetic core or permanent magnet. The solenoids 71 can actuate the distal mount 33 to helically move along a thread 6 of the tubular member 9 such that the distal mount 33 is guided along a defined trajectory when converting the sealing elements 21, 22 from the open configuration 301 to the closed configuration and vice versa. The thread 6 has a minor protrusion 61 at a defined position which provides an additional resistance when the distal mount 33 is guided along the thread 6. Such a protrusion 61 allows the sealing element 21, 22 to be held more rigidly in a certain position, for example in order to receive surgical instruments, in particular catheters or guidewires, with a precise fit. The proximal mount 31 and the distal mount 33 are held apart by a restoring force of an elastic spring 711. Thereby, the sealing elements 21, 22 assume the open configuration 301 as a default state in the absence of any actuating forces. In turn, when the solenoid 71 is actuated, the distal mount 33 performs a helical movement in a proximal direction, guiding the distal mount 33 along the thread 6.

Figure 4 shows a longitudinal section of a fourth embodiment of a surgical access port 101 with a sealing, unit 2 having two sealing elements 21, 22. The sealing elements 21, 22 are connected to a volume adjustment unit 8 which can supply a pressure tight fluid chamber 81 with fluid from a fluid reservoir (not shown in Fig. 4) by opening/closing remote controlled valves 82. The valves 82 can be controlled independently from each other by a controller (not shown in Fig. 4). In Fig. 4 the fluid chambers 81 have a radially inwardly directed and essentially semi-circular shape when fully inflated.

In Fig. 4 the first sealing element 21 and the second sealing element 22 are supplied by the same fluid reservoir. In Fig. 4 the sealing elements 21, 22 are in an intermediate configuration 302 such that the cross-section 4 of the apertures 211, 221 are still not fully closed. By supplying additional pressurized fluid through the valves 82 the fluid chambers 81 delimited by a flexible and elastic elastomer can be inflated and converted to the closed configuration. If the pressure within the fluid chambers 81 is instead decreased, the sealing elements 21, 22 are converted to the open configuration.

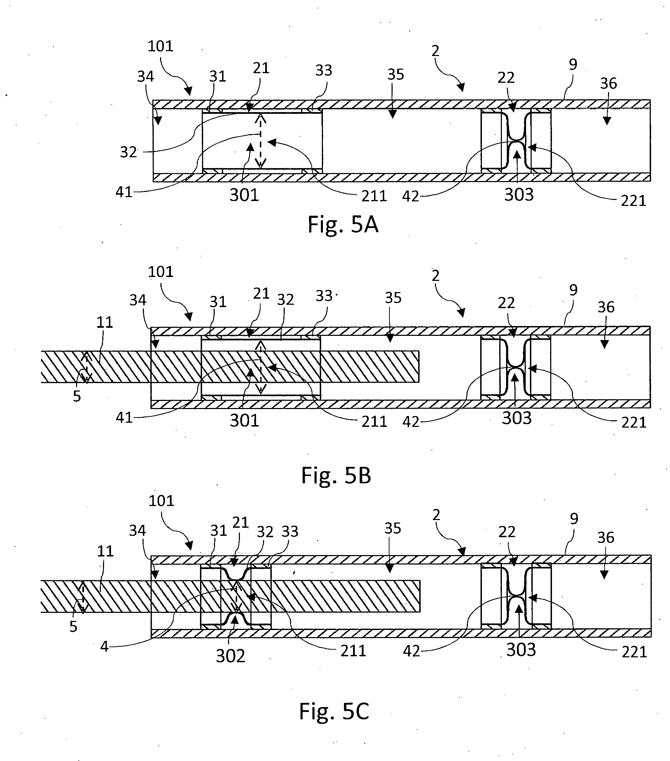

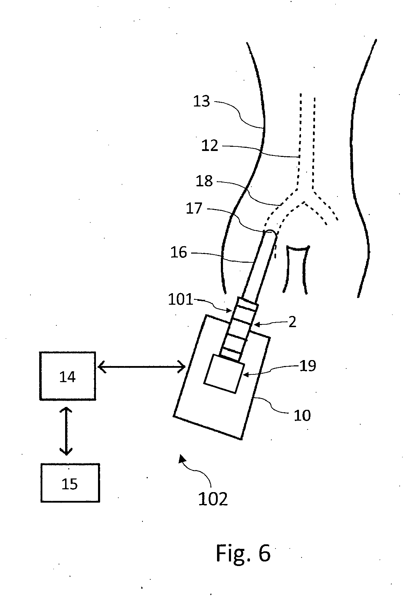

Figures 5A to 5F show a longitudinal section of the embodiment of the surgical access port 101according to Fig. 2A and Fig. 2B during insertion of a surgical instrument 11 through the sealing unit 2. The sealing unit 2 comprises a first sealing element 21 and a second sealing element 22 which can be operated by a controller 10 (see Fig. 6) independently from each other. Thereby, direct non-sterile manipulation of the surgical access port 101 can be avoided. Furthermore, when combined with an instrument inserter 19 (see Fig. 6) or robot, an automatic insertion of surgical instrument 11 through the surgical access port 101 in an endovascular surgery in an artery of a patient is also possible.

In Fig. 5A the first sealing element 21 is in an open configuration 301 such that the aperture 211 has a maximum cross-section 41. A proximal mount 31 and a distal mount 33 of the first sealing element 21 in the open configuration 301 are spaced apart such that a membrane 32 which is connected to both mounts 31, 33 assumes an essentially cylindrical shape.

The proximal mounts 31 of the sealing elements 21, 22 in Fig. 5A to Fig. 5F are fixedly attached to a continuous tubular member 9 of the surgical access port 101.

The distal mounts 33 of the sealing elements 21, 22 are movably attached such that they can perform a helical relative movement towards the corresponding proximal mount 31.

In Fig. 5A to Fig. 5C the second sealing element 22 is in a closed configuration 303 such that the aperture 221 of the second sealing element 22 has a minimum cross-section 42, so that the second sealing element 22 is pressure tight against a distal side 36.

However, in Fig. 5A and Fig. 5B a proximal side 34 and an intermediate section 35 of the surgical access port 101 are fluidly connected to each other across the first sealing element 21. In the closed configuration 303 of the second sealing element 22, shown in Fig. 5A - 5C, the proximal mount 31 and the distal mount 33 of the second sealing element 22 are arranged less spaced apart than in the open configuration 301, such that the membrane 32 is curved radially inwardly. By curving radially inwardly, the opposing sides of the membrane 32 contact each other to close the second sealing element 22.

Fig. 5B shows that a surgical instrument 11 with a particular cross-sectional dimension 5 is inserted through the aperture 211 with a maximum cross-section 41 of the first sealing element 21 without contacting the membrane 32.

Fig. 5C shows that the first sealing element 21 is converted from the open configuration 301 to an intermediate configuration 302 such that the membrane 32 forms a cross-section 4 which corresponds to the cross-sectional dimension 5 of the surgical instrument 11. By enclosing the surgical instrument 11 circumferentially, the membrane 32 renders the proximal side 34 sealed from the intermediate section 35.

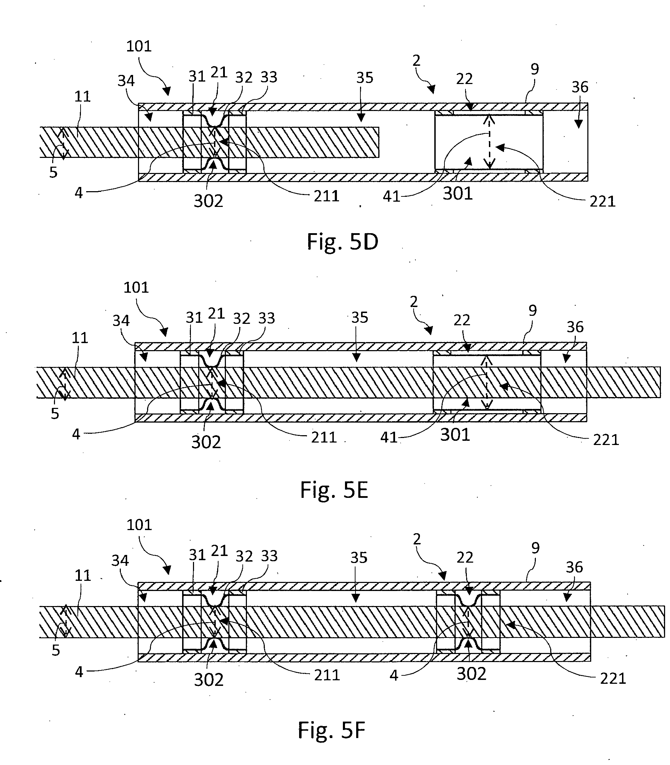

Consequently, the second sealing element 22 as shown, in Fig. 5D, can be converted to the open configuration 301 with a maximum cross-section 41 while the proximal side 34 of the surgical access port 101 is still sealed from the intermediate section 35 in order to avoid leakage of blood from the distal side 36.

Fig. 5E shows that the surgical instrument 11 is advanced through the second sealing element 22 into the artery of a patient.

Fig. 5F shows that the second sealing element 22 can be converted to the intermediate configuration 302 with a cross-section 4 corresponding to the cross-sectional dimension 5 of the surgical instrument 11 to seal the intermediate section 35 from the distal side 36.

A controller (see Fig. 6) is configured to adjust the sealing elements 21, 22 such that the friction between the surgical instrument and the membrane 32 is minimal while at the same time remaining leak tight.

Fig. 6 shows a schematic arrangement of a system 102 comprising a surgical access port 101 connected to a pressurized anatomical region 12 of a patient 13. The system 102 comprises a controller 10 which can be operated using a user interface 14 of the system 102. The user interface 14 may be operated by an operator, e.g., a surgeon 15, to perform an endovascular surgery. In particular, the user interface 14 allows to set a mode of operation or settings for frictional resistance applied by the sealing unit to surgical instruments in the surgical access port 101. The mode of operation or settings of the surgical access port 101 are then carried out by the controller 10 which is in data communication with the user interface 14. The controller 10 can further be configured to operate an instrument inserter 19. The instrument inserter 19 can e.g. be configured to frictionally engage a surgical instrument and advance the surgical instrument in a longitudinal direction of the surgical access port 101 through the sealing unit 2. Thus, the operation of the instrument inserter 19 can be precisely coordinated in terms of a position of the surgical instrument at a particular time with the operation of the sealing unit 2 by the controller 10.

In Fig. 6 a sheath 16 of the system is connected to the surgical access port 101. The sheath 16 is percutaneously introduced into a femoral artery 18 of the patient 13 through an incision 17 to establish a fluid connection to the pressurized anatomical region 12. The control of a sealing unit 2 by the controller 10 and operation of the instrument inserter 19 allows fully automated, remote controlled insertion of surgical instruments with different cross-sectional dimensions into the pressurized anatomical region 12.

## Claims

1. Surgical access port (101) for introduction of surgical instruments (11) into a pressurized anatomical region (12), wherein the surgical access port (101) comprises an elongated lumen (1) and at least one sealing unit (2) placed or placeable within the lumen (1), wherein the sealing unit (2) comprises at least one sealing element (21, 22) which is convertible from an open configuration (301) to a closed configuration (303) and vice versa,
wherein
in the closed configuration (303) an aperture (211, 221) of the sealing element (21, 22) is closed radially inward to a smaller cross-section (4), preferably a cross-section of 0.0 mm² to render the lumen (1) pressure tight,
and, wherein
in the open configuration (301) of the sealing element (21, 22) the aperture (211, 221) has a maximum cross-section (41), such that a fluid communication is established between a proximal side (34) of the sealing element (21, 22) and a distal side (36) of the sealing element (21, 22), **characterized in that**,
the sealing element (21, 22) comprises an intermediate configuration (302) between the open configuration (301). and the closed configuration (303) in which the cross-section (4) of the aperture (211, 221) is configured to receive surgical instruments (11) having a cross-sectional dimension (5), corresponding to the cross-section (4) of the aperture (211, 221), to be inserted through the sealing unit (2) into the pressurized anatomical region (12) rendering the sealing element (21, 22) pressure tight.

2. Surgical access port (101) according to claim 1, wherein the sealing element (21, 22) comprises a proximal mount (31), a distal mount (33),and a closing element (32), wherein the proximal mount (31) and the distal mount (33) are connected to the closing element (32),
wherein
the closed configuration (303) can be converted via the intermediate configuration (302) to the open configuration (301) of the sealing element (21, 22) by a relative movement of one of the proximal mount (31) and the distal mount (33) relative to the other of the proximal mount(31) and the distal mount (33).

3. Surgical access port (101) according to claim 2, wherein the relative movement of the proximal mount (31) and/or distal mount (33) is one of the following:
- A relative movement in a longitudinal direction (L) of the surgical access port (101),
- A relative movement in a rotational direction (G), preferably in a circumferential direction of the surgical access port (101), and
- A relative movement in the longitudinal direction (L) of the of the surgical access port (101) and in the rotational direction (G), preferably in the circumferential direction of the surgical access port (101).

4. Surgical access port (101) according to claim 2 or 3,
wherein the closing element (32) comprises or consists of a flexible material such as a thermoplastic material, such as silicone, latex, polyurethane, polyethylene, polyamide, polyethylene terephthalate, polyvinyl chloride, polyether block amide, and preferably forms a continuous at least partially tubular structure (321).

5. Surgical access port (101) according to one of the claims 2 to 4, wherein the surgical access port (101) comprises a guiding structure (6) for guiding at least one of the proximal mount (31) and the distal mount (33) during relative movement, preferably comprising a retaining element (61) which causes an increased resistance against the relative movement in at least a partial area of the guiding, structure (6).

6. Surgical access port (101) according to any one of the preceding claims, wherein the sealing unit (2) comprises a first sealing element (21) and a second sealing element (22) which are spaced apart from each other in the longitudinal direction (L) of the surgical access port (101).

7. Surgical access port (101) according to claim 6, wherein the first sealing element (21) and the second sealing element (22) are convertible from the closed configuration (303) to the open configuration (301) via the intermediate configuration (302) and vice versa independently from each other.

8. Surgical access port (101) according to any one of the preceding claims, wherein the sealing unit (2) has an actuation element for operating the at least one sealing element (21, 22), wherein the actuation element is one of:
- A pneumatic,
- a hydraulic,
- an electrical,
- a magnetic,
- an electromagnetic (71),
- an electromechanical, and
- a mechanical actuation element.

9. Surgical access port (101) according to claim 8, wherein the actuation element comprises an actuation guiding structure which is arranged on an outer surface of the surgical access port and preferably forms a thread shape or slots.

10. Surgical access port (101) according to any one of the preceding claims, wherein the sealing element (21, 22) comprises a volume adjustment unit (8) for operating the sealing element (21, 22) by adjusting the volume of a fluid in at least one pressure tight fluid chamber (81) of the volume adjustment unit (8),
wherein the volume adjustment unit (8) further comprises at least one fluid reservoir fluidly connected or fluidly connectable to the at least one fluid chamber (81).

11. Surgical access port (101) according to claim 10, wherein the volume adjustment unit (8) comprises at least two independently controllable valves (82) to operate at least two sealing elements (21, 22).

12. Surgical access port (101) according to any one of the preceding claims, wherein the surgical access port (101) comprises an outer monolithic tubular member (9), preferably forming a substantially continuous surface.

13. System (102) comprising a surgical access port (101) according to any one of the claims 1 to 12, wherein the system (102) comprises a controller (10) configured to operate the at least one sealing element (21, 22) of the sealing unit (2), preferably at least two sealing elements (21, 22) independently from each other.

14. System (102) comprising a surgical access port (101) according to any one of the claims 1 to 12 and at least one surgical instrument (11), preferably at least two surgical instruments (111, 112) with at least two different cross-sectional dimensions (5).

15. Method, for introduction of surgical instruments (11) of different cross-sectional dimensions (5) into a pressurized anatomical region (12) through a surgical access port (101), in particular according to one of the claims 1 - 12, the method comprising the steps of:
- Providing a second sealing element (22) in a closed configuration (303);
- Optionally converting a first sealing element (21) from a closed configuration (303) to an intermediate configuration (302) or an open configuration (301);
- Introducing the surgical instrument (11) from a proximal side (34) to an intermediate section (35) of the surgical access port (101) through a first aperture (211) of the first sealing element (21);
- Converting the first sealing element (21) from the intermediate configuration (302) or open configuration (301) such that the cross-section (4) of the first aperture (211) encloses the surgical instrument (11) in a pressure tight manner;
- Optionally converting the second sealing element (22) from the closed configuration (303) to the intermediate configuration (302) or open configuration (301);
- Introducing the surgical instrument (11) from the intermediate section (35) to the distal side (36) of the surgical access port (101) through the second aperture (221) of the second sealing element (22).
- Converting the second sealing element (22) from the intermediate configuration (302) or open configuration (301) such that the cross-section (4) of a second aperture (221) encloses the cross-sectional dimension (5) of the surgical instrument (11) in a pressure tight manner.
